(19) Europäisches Patentamt
European Patent Office
Office européen des brevets

(11) **EP 2 574 335 B1**

(12) **EUROPEAN PATENT SPECIFICATION**

(45) Date of publication and mention
of the grant of the patent:
**03.02.2016 Bulletin 2016/05**

(51) Int Cl.:
***A61K 9/70*** *(2006.01)*

(21) Application number: **12186402.9**

(22) Date of filing: **27.09.2012**

(54) **Manufacturing method of patch preparation**

Herstellungsverfahren zur Pflasterherstellung

Procédé de fabrication d'une préparation de patch

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.09.2011 JP 2011214850**

(43) Date of publication of application:
**03.04.2013 Bulletin 2013/14**

(73) Proprietor: **Nitto Denko Corporation
Osaka 567-8680 (JP)**

(72) Inventors:
• **Sakamoto, Sachiko
Osaka, 567-8680 (JP)**
• **Hanatani, Akinori
Osaka, 567-8680 (JP)**
• **Okazaki, Arimichi
Osaka, 567-8680 (JP)**
• **Mukobata, Tsuyoshi
Osaka, 567-8680 (JP)**

(74) Representative: **Duckworth, Timothy John
J A Kemp
14 South Square
Gray's Inn
London WC1R 5JJ (GB)**

(56) References cited:
**EP-A1- 2 377 515      EP-A2- 0 304 227
WO-A1-96/19394      WO-A1-2004/058247**

**Description**

The present invention relates to a manufacturing method of a patch preparation comprising a support and an adhesive layer containing a drug, which is provided on one surface of the support.

BACKGROUND OF THE INVENTION

Transdermal administration of a drug can avoid first-pass metabolism of the drug in the liver and various side effects, and enables long-term administration of the drug in a sustained manner. Particularly, a patch preparation containing a drug in an adhesive layer has been actively developed, since an administration method thereof is easy and the dose can be controlled strictly.

As a method for efficient absorption of a drug from a patch preparation into the body through the skin, a method of increasing the concentration of a drug in an adhesive layer, and a method of increasing the area and thickness of an adhesive layer are known. However, as the concentration of a drug contained in an adhesive layer increases, crystals of the drug tend to be formed in the adhesive layer, and when the area and thickness of the adhesive layer of a patch preparation are increased, problems occur such as lower handling property when the patch preparation is used, increased unpleasant feeling during adhesion to the skin.

When a small amount of crystal of the drug is precipitated in an adhesive layer, an adverse influence is not exerted on the usefulness of a patch preparation; however, it may give rise to a concern of the patients about degradation of the quality, and may induce inconveniences such as hesitation in using the patch preparation and the like. When a large amount of crystal of the drug is precipitated, releasability of the drug changes, and when the surface of an adhesive layer is covered with the crystal, the area having adhesiveness decreases, and therefore, adhesiveness to the skin decreases, and the patch preparation may not stand long-term adhesion.

JP-A-2006-513195 and JP-B-3566301 disclose a heat treatment of an adhesive layer containing a drug at a supersaturation concentration to manufacture a patch preparation suppressed in crystallization. However, since the temperature of the heating step in the manufacturing method is not less than the melting point of the drug, denaturation of the drug is feared. Moreover, in the method described in said patent, a laminate sheet with a laminate constitution of support/drug-containing adhesive layer/release liner is cut into a patch preparation with a given shape. According to this method, since the laminate sheet after cutting out the patch preparation is generally discarded as a waste, manufacturing loss is involved. Moreover, since the laminate sheet including the manufacturing loss is manufactured, unnecessary material costs are also involved.

SUMMARY OF THE INVENTION .

The present invention has been made in view of the above-mentioned situation, and aims to solve the problem of the provision of an economical manufacturing, method of a patch preparation that decreases the manufacturing loss.

In addition, the present invention aims to provide a manufacturing method of a patch preparation that can afford a sufficiently high skin permeation amount of a drug (that is, shows superior releasability of a drug) even without increasing the area and thickness of an adhesive layer, wherein precipitation of crystal of the drug is extremely difficult.

The present inventors have conducted intensive studies in an attempt to solve the aforementioned problems and found that the precipitation of crystal of the drug in a drug-containing adhesive layer is greatly influenced by a physical stimulation applied to the drug-containing adhesive layer. They have conducted further studies to reduce physical stimulation applied to the drug-containing adhesive layer as much as possible, and found that, according to the manufacturing method below, a patch preparation can be manufactured without applying the physical stimulation which may cause the precipitation of crystal of the drug to the drug-containing adhesive layer to complete the present invention.

A method of manufacturing a patch preparation comprising:

pouring a composition for forming an adhesive layer into a container having a concave part with a shape corresponding to a patch preparation to be manufactured, wherein the composition contains a drug, an adhesive polymer and an organic solvent;
removing the organic solvent from the composition through drying to form a drug-containing adhesive layer in the concave part; and
laminating a support on the drug-containing adhesive layer to give the patch preparation comprising the support and the drug-containing adhesive layer formed on one surface of the support.

Accordingly, the present invention is as follows.

[1] A method of manufacturing a patch preparation comprising:

pouring a composition for forming an adhesive layer into a container having a concave part with a shape corresponding to a patch preparation to be manufactured, wherein the composition contains a drug, an adhesive polymer and an organic solvent, wherein the depth of the concave part is not less than 110% of the thickness of the drug-containing adhesive layer of the patch preparation to be manufactured, and the difference between the depth of the concave part and thickness of the drug-containing adhesive layer (depth of concave part minus thickness of drug-containing adhesive layer) is not more than 20mm;

removing the organic solvent from the composition through drying to form the drug-containing adhesive layer in the concave part; and

laminating a support on the drug-containing adhesive layer to give the patch preparation comprising the support and the drug-containing adhesive layer formed on one surface of the support.

[2] The method of the above-mentioned [1], wherein the concave part of the container has a peel-treated inner surface.

[3] The method of the above-mentioned [1] or [2], wherein the drug is a solid at room temperature.

[4] The method of the above-mentioned [1] or [2], wherein the drug does not have a crystallization peak in a DSC measurement.

[5] The method of the above-mentioned [1] or [2], wherein the composition for forming an adhesive layer has a drug concentration adjusted such that the drug dissolved in the drug-containing adhesive layer formed in the concave part does not form a crystal immediately after application of physical stimulation to the adhesive layer, but forms a crystal when the preparation is further preserved.

[6] A method of manufacturing a patch preparation package, comprising manufacturing a patch preparation according to the method of any one of the above-mentioned [1] to [5], housing the patch preparation in a container having a flange formed on the periphery of an opening, and adhering a cap material to the flange to tightly seal the container.

The present invention can provide an economical manufacturing method of a patch preparation, which method does not involve manufacturing of a portion to be discarded.

In addition, the present invention can provide a patch preparation having a drug-containing adhesive layer with a suitable area and thickness that do not degrade handling property of the patch preparation, which shows sufficiently high drug permeability (namely, high releasability), is substantially free of drug precipitation after preservation, and maintains adhesiveness to the skin, permitting long-term adhesion.

BRIEF DESCRIPTION OF THE DRAWINGS

Fig. 1 is a schematic explanatory view of the manufacturing method of the patch preparation in a first embodiment of the present invention.

Fig. 2 is a perspective view of the container in Fig. 1.

Fig. 3 is a schematic explanatory view of the manufacturing method of the patch preparation in a second embodiment of the present invention.

Fig. 4 is a perspective view of the container in Fig. 3.

In the Figures, 1 is a patch preparation, 2 is a support, 3 is a drug-containing adhesive layer, 11 is a container, 12 is a concave part, 12A is a first concave part, and 12B is a second concave part.

DESCRIPTION OF EMBODIMENTS

The present invention is explained in the following by referring to the embodiments thereof.

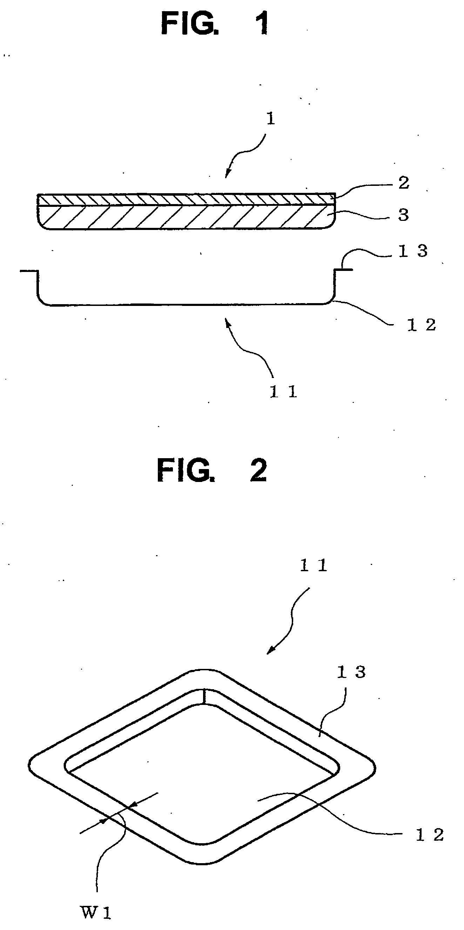

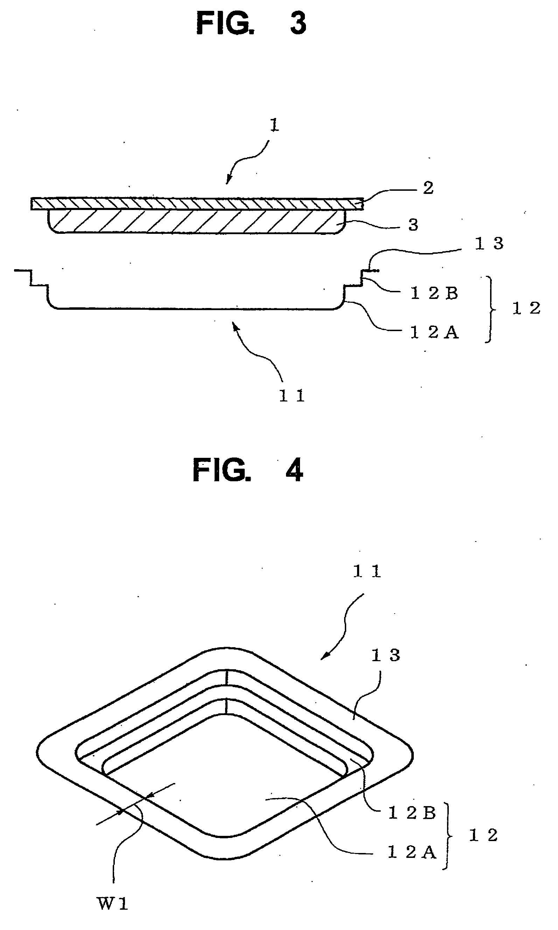

The manufacturing method of the patch preparation of the present invention (hereinafter sometimes to be abbreviated as "the method of the present invention") mainly comprises, as shown in Figs. 1 and 3, pouring a composition for forming an adhesive layer (not shown), which composition containing a drug, an adhesive polymer and an organic solvent, into a container 11 having a concave part 12 with a shape corresponding to a patch preparation 1 (the patch preparation to be manufactured), removing the the organic solvent from the composition through drying, thereby forming a drug-containing adhesive layer (hereinafter sometimes to be simply referred to as "adhesive layer") 3 in the concave part 12, and laminating a support 2 on the drug-containing adhesive layer 3.

In the method of the present invention, the "concave part 12 with a shape corresponding to a patch preparation to be manufactured" in the container 11 means a concave part having a shape and size determined such that at least the drug-containing adhesive layer 3 having a given flat planar shape and thickness of a patch preparation to be manufactured can be formed after removing the organic solvent from the composition for forming an adhesive layer through drying inside the container. The concave part 12 only needs to have a depth sufficient to contain at least the drug-containing adhesive layer 3. It may be a depth permitting lamination of the support 2 on the drug-containing adhesive layer 3 to be performed inside the container. Since the volume of the composition for forming an adhesive layer, which is poured into the concave part 12, decreases by removing the organic solvent from the composition through drying, the thickness of the drug-containing adhesive layer formed in the concave part 12 is lower than that of the composition for forming an adhesive layer, which is poured into the concave part 12. Hence, the depth of the concave part 12 needs to be, at minimum, about 110% of the thickness of the drug-containing adhesive layer 3 of a patch preparation to be manufactured. When the depth of the concave part 12 is too large after forming the drug-containing adhesive layer 3, an operation to take out the drug-containing adhesive layer 3 from the concave part 12 and/or an operation to laminate the support 2 on the drug-containing adhesive layer 3 in the concave part 12 become(s) difficult. Thus, the difference between the depth of the concave part 12 and the thickness of the drug-containing adhesive layer 3 (depth of concave part minus thickness of drug-containing adhesive layer) is not more than 20 mm.

In the case of container 11 shown in Figs. 1 and 2, the concave part 12 is formed with a single concave part. When the support 2 is laminated on the drug-containing adhesive layer 3 in the concave part 12, a patch preparation 1, wherein the size and shape of the flat plane of the support 2 and the drug-containing adhesive layer 3 are substantially the same, is manufactured.

On the other hand, the container 11 shown in Figs. 3 and 4 has a concave part with a two-stage structure consisting of a first concave part 12A to be the mold for forming the drug-containing adhesive layer 3, and a second concave part 12B having a shape corresponding to the support 2, which has a flat plane size larger than that of the first concave part 12A. Using the container 11 shown in Figs. 3 and 4, a patch preparation 1 wherein the size of the flat plane of the support 2 is than that of the drug-containing adhesive layer 3 can be manufactured by pouring a composition for forming an adhesive layer (not shown), containing a drug, an adhesive polymer and an organic solvent, into the first concave part 12A of the container 11, removing the organic solvent from the composition through drying to form the drug-containing adhesive layer 3 in the first concave part 12A, inserting the support 2 into the second concave part 12B of the container, and laminating the support 2 on the drug-containing adhesive layer 3.

In the present invention, while the flat plane size of the concave part 12 to be formed in the container 11 is not particularly limited, in the case of the container 11 shown in Figs. 1 and 2, the flat plane size of the concave part 12 is generally about 0.5 - 80 cm$^2$, preferably 0.5 - 60 cm$^2$.

In addition, in the case of the container 11 shown in Figs. 3 and 4, the flat plane size of the first concave part 12A is generally about 0.5 - 80 cm$^2$, preferably 0.5 - 60 cm$^2$. The flat plane size of the second concave part 12B that receives the support 2 is generally about 0.5 - 120 cm$^2$, preferably 0.5 - 100 cm$^2$. The flat plane size of the first concave part 12A and the second concave part 12B is preferably determined such that the second concave part 12B is larger by 0.5 - 10 mm in the entire circumference of the first concave part 12A. In this way, a patch preparation having a tab with a sufficient and appropriate width, which is ensured on the periphery of the support 2, can be manufactured easily.

The container 11 in the present invention preferably becomes a mold for forming the drug-containing adhesive layer 3 in the manufacturing of a patch preparation, and has shape retainability and strength capable of protecting the drug-containing adhesive layer until use of the patch preparation. Accordingly, while the container is not particularly limited, preferred is one obtained by forming a plastic sheet conventionally used as a blister bottom material in producing a blister package of a patch preparation, by a known sheet molding method such as vacuum molding method, pressure molding method, vacuum-pressure molding method, compression molding method and the like, into a container having a concave part with a shape corresponding to a patch preparation. As such plastic sheet, general purpose plastic products such as polyethylene, poly(ethylene terephthalate), polystyrene, polycarbonate, polypropylene, polyamide, polyester, polyvinyl chloride and the like can be used. The sheet may be a single layer sheet made of one or more kinds of plastic, or a laminate sheet having two or more layers each made of different plastic. To impart sufficient gas barrier property to the sheet, moreover, a gas barrier sheet obtained by laminating an ethylene-vinyl acetate copolymer saponified product, acrylonitrile resin, polyvinylidene chloride and the like on the above-mentioned general purpose plastic sheet can be used. The acrylonitrile resin in this context is not particularly limited as long as not less than 50 wt% of the total weight of the resin is constituted with an acrylonitrile component. From the aspects of flexibility and heat sealing property, a copolymer containing acrylonitrile as the main structural unit is preferable, and the copolymer preferably contains, as the structural unit, at least acrylonitrile, a rubber component such as butadiene and the like, and/or (meth)acrylic acid alkyl ester wherein the alkyl group has 1 - 6 carbon atoms. In said copolymer, the content of acrylonitrile is preferably 50 - 90 wt%, and a particularly preferable copolymerization composition is acrylonitrile (50 - 90 wt%), rubber component such as butadiene and the like (2 - 12 wt%), and (meth)acrylic acid alkyl ester wherein the alkyl group has 1 - 6 carbon atoms (8 - 38 wt%). The gas barrier sheet is particularly preferably a laminate sheet obtained by laminating a poly(ethylene terephthalate) sheet simultaneously showing shape retainability, strength, flexibility and heat sealing property, and an acrylonitrile resin sheet. When the container requires light blocking property, a laminate sheet obtained by further laminating a film having light blocking property-such as a metal foil and the like on the above-mentioned general purpose plastic sheet or gas barrier sheet, and the like can be used. In addition, a sheet obtained by further vapor-depositing a metal on the sheet explained above may be used.

In consideration of the removal of the organic solvent from the composition for forming an adhesive layer during formation of a drug-containing adhesive layer in the container, heating to introduce a crosslinking structure into the adhesive layer and the like, it is preferred that the plastic sheet constituting the container not melt or deform by heating. Thus, as the plastic sheet, a plastic sheet having a glass transition temperature in a DSC (differential scanning calorimeter) measurement higher than the heating temperature in forming the drug-containing adhesive layer is preferably selected.

In addition, as mentioned below, the container in the present invention may be directly used as a blister (package container) after manufacturing of the patch preparation. In such case, to adhere a cover sheet for sealing the container, the surface to be adhered to the cover sheet is preferably coated with, as a thermally adhesive material, for example, a polyolefin hot-melt adhesive, a sealant film such as polyacrylonitrile and the like, and the like.

While the thickness of the sheet constituting the container is not particularly limited, a thickness capable of ensuring the strength to prevent the concave part of the container from being excessively deformed during manufacturing of the patch preparation is preferable. Furthermore, in consideration of the continuous manufacturing of the package of the patch preparation by directly using the container as a blister (package container) after manufacturing of the patch preparation as mentioned below, and the like, it is necessary to ensure a thickness that prevents permeation of a drug in the patch preparation. From such aspect, the total thickness of the above-mentioned sheet is generally 10 - 1000 $\mu$m, preferably 25 - 500 $\mu$m.

The container 11 in the present invention is preferably applied with a peel treatment (specifically, surface treatment with silicone release agent, fluorine release agent, wax and the like) of the inside of the concave part 12, so that the drug-containing adhesive layer 3 formed in the concave part 12 can be easily detached from the inside of the concave part 12.

Moreover, in consideration of the continuous manufacturing of the package of the patch preparation by directly using the container 11 as a blister (package container) after manufacturing of the patch preparation, the container 11 preferably has a constitution wherein a flange (projected piece) 13 is formed on the periphery of the opening thereof, as shown in Figs. 1 - 4. With this constitution, a package can be easily manufactured by adhering a cover sheet (not shown) for tightly sealing the container to said flange (projected piece) 13, and a sealed structure of the package can be certainly obtained. While the width (W1 in Figs. 2 and 4) of the flange (projected piece) 13 is not particularly limited, it is generally about 1 - 50 mm, preferably about 1 - 20 mm.

[Composition for forming adhesive layer]

In the present invention, the composition for forming an adhesive layer is a composition containing at least an adhesive polymer, a drug and an organic solvent, and further, a tackifier, an organic liquid component, a crosslinking agent and the like as necessary. The composition for forming an adhesive layer is generally prepared as a solution having a concentration of the essential components forming the adhesive layer such as adhesive polymer, drug and the like, or the total concentration of the essential components added with a tackifier, a crosslinking agent, an organic liquid component and the like, of not less than about 15 wt% and not more than about 90 wt%.

The above-mentioned drug may be any as long as it can be transdermally absorbed and administered to mammals such as human and the like through the skin thereof, and a known drug conventionally used as a drug for a patch preparation can be used without limitation. Specific examples of such drug include adrenocorticosteroidal agents, nonsteroidal antiinflammatory agents, antirheumatic agents, sleeping agents, antipsychotic agents, antidepressants, psychostimulant agents, anxiolytic agents, antiepileptic agents, therapeutic agents for migraine, anti-Parkinsonian agents, ameliorants for cerebral circulation/metabolism, anti-dementia agents, autonomic drugs, muscle relaxants, hypotensive agents, diuretics, hypoglycemic agents, antihyperlipidemic agents, antipodagrics, systemic anesthetics, local anesthetics, antibacterial agents, antifungal agents, antiviral agents, antiparasitic agents, vitamin agents, antianginal agents, vasodilators, antiarrhythmics, antihistaminic agents, mediator release inhibitors, leukotriene antagonists, female hormone drugs, thyroid hormone drugs, antithyroid agents, antiemetic agents, antidizzying agents, bronchodilators, antitussives, expectorants, smoking renunciation aids and the like.

The organic solvent is an organic compound which is liquid at room temperature (25°C) and dissolves an adhesive polymer, a drug and the like, and preferably has a boiling point of less than 150°C at normal pressure so that it can be sufficiently removed through drying when forming the adhesive layer. Therefore, methanol, ethanol, benzene, n-hexane, ethyl acetate, acetone, toluene, xylene, heptane, isopropyl alcohol and the like are preferable.

As mentioned above, in the container 11, the concave part 12 with a shape at least corresponding to the drug-containing adhesive layer 3 of the patch preparation 1 (a patch preparation to be manufactured) is formed, and a drug containing adhesive layer 3 can be formed in the concave part 12 by pouring a composition for forming an adhesive layer into the container 11, and removing the organic solvent from the composition through drying. The drug-containing adhesive layer 3 of the patch preparation to be manufactured in the present invention is preferably a non-hydroscopic adhesive layer from the aspects of skin adhesiveness. Therefore, a composition for forming an adhesive layer is prepared using an organic solvent. The "non-hydroscopic adhesive layer" means an adhesive layer having a water content of less than 10 wt%, preferably less than 5 wt%, more preferably less than 3 wt%, most preferably less than 2 wt%.

The adhesive polymer is not particularly limited, and acrylic polymers including (meth)acrylic acid ester polymer; rubber polymers such as styrene-isoprene-styrene block copolymer, styrene-butadiene-styrene block copolymer, polyisoprene, polyisobutylene, polybutadiene and the like; silicone polymers such as silicone rubber, dimethylsiloxane base, diphenylsiloxane base and the like; vinyl ether polymers such as polyvinyl methyl ether, polyvinyl ethyl ether, polyvinyl isobutyl ether and the like; vinyl ester polymers such as vinyl acetate-ethylene copolymer and the like; ester polymers comprising a carboxylic acid component such as dimethyl terephthalate, dimethyl isophthalate, dimethyl phthalate and the like, and a polyvalent alcohol component such as ethylene glycol and the like; and the like can be mentioned.

The acrylic polymer is preferably one obtained by copolymerizing (meth)acrylic acid alkyl ester as a main component with a functional monomer. To be specific, a copolymer containing 50 - 99 wt% (preferably 60 - 95 wt%) of a monomer component comprised of (meth)acrylic acid alkyl ester, and the remaining monomer component comprised of a functional monomer is preferable. The main component here means a monomer component contained in a proportion of not less than 50 wt% of the total weight of monomer component constituting the copolymer.

The (meth)acrylic acid alkyl ester (hereinafter to be also referred to as a main component monomer) is generally comprised of a straight chain or branched alkyl group wherein the alkyl group has 4 - 13 carbon atoms (e.g., butyl, pentyl, hexyl, heptyl, octyl, 2-ethylhexyl, nonyl, decyl, undecy-1, dodecyl, tridecyl and the like), and one or more kinds thereof are used.

The functional monomer has at least one unsaturated double bond relating to the copolymerization reaction in a molecule, as well as a functional group in the side chain. Examples thereof include carboxyl group-containing monomers such as (meth)acrylic acid, itaconic acid, maleic acid, maleic anhydride; hydroxyl group-containing monomers such as (meth)acrylic acid hydroxyethyl ester, (meth)acrylic acid hydroxypropyl ester; sulfo group-containing monomers such as styrene sulfonic acid, allyl sulfonic acid, sulfopropyl(meth)acrylate, (meth)acryloyloxynaphthalene sulfonic acid, acrylamide methylpropane sulfonic acid; amino group-containing monomers such as (meth)acrylic acid aminoethyl ester, (meth)acrylic acid dimethylaminoethyl ester, (meth)acrylic acid tert-butylaminoethyl ester; amide group-containing monomers such as (meth)acrylamide, dimethyl(meth)acrylamide, N-methylol(meth)acrylamide, N-methylolpropane(meth)acrylamide, N-vinylacetamide; and alkoxyl group-containing monomers such as (meth)acrylic acid methoxyethyl ester, (meth)acrylic acid ethoxyethyl ester, (meth)acrylic acid methoxyethylene glycol ester, (meth)acrylic acid methoxydiethylene glycol ester, (meth)acrylic acid methoxypolyethylene glycol ester, (meth)acrylic acid methoxypolypropylene glycol ester, (meth)acrylic acid tetrahydrofuryl ester.

One or more kinds of the functional monomers can be used. Among those, carboxyl group-containing monomer is preferable, and (meth)acrylic acid is particularly preferable, from the aspects of the pressure-sensitive adhesiveness and cohesiveness of an adhesive layer, releasability of a drug contained in an adhesive layer and the like.

As an acrylic polymer, one obtained by copolymerization of the above-mentioned copolymer of (meth)acrylic acid alkyl ester (main component monomer) and a functional monomer, with other monomer can also be used.

Examples of other monomer include (meth)acrylonitrile, vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole and the like. One or more kinds thereof can be used.

The amount of other monomer to be used is generally preferably about 0 - 40 wt%, more preferably about 10 - 30 wt%, relative to the total weight of (meth)acrylic acid alkyl ester (main component monomer) and a functional monomer.

Preferable specific examples of the acrylic polymer include a terpolymer of 2-ethylhexyl acrylate as (meth)acrylic acid alkyl ester, acrylic acid, and N-vinyl-2-pyrrolidone, since it shows good adhesiveness to the human skin, and adhesion and detachment can be easily repeated, with more preference given to a copolymer of 2-ethylhexyl acrylate, acrylic acid, and N-vinyl-2-pyrrolidone copolymerized at a weight ratio of 40 - 99.8:0.1 - 10:0.1 - 50.

The rubber polymer is preferably a rubber polymer comprising at least one kind selected from polyisobutylene, polyisoprene and styrene-diene-styrene block copolymer (styrene-butadiene-styrene block copolymer (SBS), styrene-isoprene-styrene block copolymer (SIS) etc.) as a main component. Since drug stability is high, and necessary adhesive force and cohesion strength can be simultaneously achieved, a mixture of a high-molecular-weight polyisobutylene having a viscosity average molecular weight of 500,000 - 2,100,000, and a low-molecular-weight polyisobutylene having a viscosity average molecular weight of 10,000 - 200,000 at a weight ratio of 95:5 - 5:95 is particularly preferable.

The viscosity average molecular weight here is obtained by calculating the Staudinger index ($J_0$) according to the following Schulz-Blaschke equation from the flow time of capillary of the Ubbelohde's viscometer at 20°C, and applying the $J_0$ value to the following equations.

[formula 1]

$$J_0 = \eta_{sp}/\{c(1+0.31\eta_{sp})\} \quad \text{(Schulz-Blaschke equation)}$$

$$\eta_{sp} = t/t_0 - 1$$

t: flow time of solution (according to Hagenbach-couette correction)
$t_0$: flow time of solvent (according to Hagenbach-couette correction)
c: concentration of solution (g/cm$^3$)

$$J_0 = 3.06 \times 10^{-2} \, Mv^{0.65}$$

Mv: viscosity average molecular weight

When a rubber polymer is used as an adhesive polymer, a composition for forming an adhesive layer preferably further contain a tackifier since an adhesive layer can have adhesiveness at ambient temperature. As the tackifier, one known in the technical field can be appropriately selected and used. Examples of the tackifier include petroleum resin (e.g., aromatic petroleum resin, aliphatic petroleum resin and the like), terpene resin, rosin resin, coumarone indene resin, styrene resin (e.g., styrene resin, poly($\alpha$-methylstyrene) and the like), hydrogenated petroleum resin (e.g., alicyclic saturated hydrocarbon resin and the like) and the like. Of these, alicyclic saturated hydrocarbon resin is preferable, since stability of the drug is improved. One or more kinds of tackifier can be used in combination, and the amount of the tackifier is generally 33 - 300 wt%, preferably 50 - 200 wt%, of the total weight of the rubber polymer.

When desired, a composition for forming an adhesive layer can contain an organic liquid component to control adhesiveness of a drug-containing adhesive layer or enhance transdermal absorption of a drug and the like. The organic liquid component is an organic compound which is liquid at room temperature (25°C) and shows an action to plasticize an adhesive layer. The organic liquid component preferably has a boiling point at normal pressure of not less than 150°C so that it can sufficiently remain in the adhesive layer when the organic solvent in the composition for forming an adhesive layer is removed through drying by heating to form an adhesive layer. Thus, preferable specific examples of the organic liquid component include higher alcohols such as oleyl alcohol, octyldodecanol; glycols such as ethylene glycol, diethylene glycol, triethylene glycol, propylene glycol, polyethylene glycol, polypropylene glycol; higher fatty acids such as caprylic acid, oleic acid; fats and oils such as olive oil, castor oil, squalene, lanolin; liquid surfactant; plasticizers such as diisopropyl adipate, phthalic acid ester, diethyl sebacate, triethyl citrate, tributyl acetylcitrate; hydrocarbons such as squalene, liquid paraffin; ethoxylated stearyl alcohol; fatty acid ester; fatty acid ester of glycerol and the like. One or more kinds of the organic liquid components can be used.

Of these, fatty acid ester, fatty acid ester of glycerol (mono-, di-, or triglyceride), octyldodecanol, tributyl acetylcitrate, liquid paraffin and the like are particularly preferable.

As the fatty acid ester, fatty acid ester comprised of higher fatty acid preferably having 12 - 16, more preferably 12 - 14, carbon atoms and lower monovalent alcohol preferably having 1 - 4 carbon atoms is preferably used to maintain compatibility with an adhesive polymer in a drug-containing adhesive layer, and to prevent volatilization, together with an organic solvent, due to drying under heating during the manufacturing step of a patch preparation to remove the organic solvent in a composition for forming an adhesive layer. As the higher fatty acid having 12 - 16 carbon atoms, lauric acid, myristic acid, palmitic acid and the like can be mentioned, and as the lower monovalent alcohol having 1 - 4 carbon atoms, methyl alcohol, ethyl alcohol, propyl alcohol, isopropyl alcohol and the like can be mentioned.

The fatty acid ester of glycerol (mono-, di-, or triglyceride) is preferably medium-chain (8 - 12 carbon atoms) fatty acid ester of glycerol, or may be either monoglyceride, diglyceride or triglyceride, or a mixture of two or more kinds thereof, with preference given to triglyceride. As the medium-chain triglyceride, triglyceride wherein only one kind of medium-chain fatty acid is ester bonded to glycerol (e.g., caprylic acid triglyceride wherein medium-chain fatty acid ester bonded to glycerol is caprylic acid alone, capric acid triglyceride wherein medium-chain fatty acid ester bonded to glycerol is capric acid alone and the like) may be used, or triglyceride wherein plural kinds of medium-chain fatty acids are ester bonded to glycerol (e.g., (caprylic acid, capric acid) triglyceride wherein the medium-chain fatty acid esters bonded to glycerol are caprylic acid and capric acid, (caprylic acid, capric acid, lauric acid) triglyceride wherein the medium-chain fatty acid esters bonded to glycerol are caprylic acid, capric acid and lauric acid and the like) may be used.

The drug-containing adhesive layer of the patch preparation is preferably a crosslinked adhesive layer, from the aspects of suitable skin adhesiveness and prevention of adhesive residue on detachment. Therefore, a composition for forming an adhesive layer may contain various crosslinking agents such as isocyanate compounds (e.g., trifunctional isocyanates and the like), organic peroxide, organometallic salt, metal alcoholate, metal chelate compound, polyfunctional compound (polyfunctional monomers for internal crosslinking such as polyfunctional external crosslinking agents, diacrylate, dimethacrylate). When a composition for forming an adhesive layer, which contains such various crosslinking agents, is used, a crosslinking treatment (chemical crosslinking treatment) is simultaneously performed when the organic solvent in the composition for forming an adhesive layer is removed by drying by heating, and a drug-containing adhesive layer having a crosslinking structure can be formed. When a crosslinking structure is introduced into a drug-containing adhesive layer by a physical crosslinking treatment, the organic solvent in the composition for forming an adhesive layer is removed through drying in the concave part 12 of the container 11 to form a drug-containing adhesive layer, and the layer is subjected to irradiation such as UV irradiation, electron beam irradiation and the like.

[Manufacturing of patch preparation]

First, a composition for forming an adhesive layer is prepared. That is, an adhesive polymer and a drug are added, together with other additives such as organic liquid component as necessary, to an organic solvent and the mixture is sufficiently mixed until it becomes uniform. When a crosslinking agent is added, it is added to the mixture and sufficiently mixed. Where necessary, an organic solvent may be added with a crosslinking agent and mixed. The thus-obtained mixture is used as a composition for forming an adhesive layer. The composition for forming an adhesive layer is prepared such that dissolves the drug in the drug-containing adhesive layer 3 formed in the concave part 12 of the container 11 in the following formation step of a drug-containing adhesive layer and the drug concentration of the adhesive layer is 100 - 300% of the saturation concentration of the drug in the adhesive layer.

Next, the composition for forming an adhesive layer is poured into the concave part 12 of the container 11, and the organic solvent in the composition for forming an adhesive layer is removed through drying to form a drug-containing adhesive layer 3. The composition for forming an adhesive layer can be poured into the container 11 (concave part 12) by, for example, a method of pouring a given amount of the composition for forming an adhesive layer into the concave part of each one container, a method including forming plural concave parts on a sheet (sheet with plural container compartments), pouring the composition for forming an adhesive layer, and removing the abundant composition left outside the concave parts to dispense a given amount of the composition for forming an adhesive layer into individual containers and the like. The organic solvent in the composition for forming an adhesive layer can be removed through drying by air drying including standing the container at room temperature to allow volatilization of the organic solvent, or drying by heating including placing the container 11 in a heating environment to allow volatilization (evaporation) of the organic solvent. In this case, the heating temperature is less than the glass transition temperature of the plastic sheet constituting the container 11, which is generally not less than 25°C and less than 100°C, preferably not less than 25°C and less than 90°C, more preferably not less than 25°C and less than 80°C.

When a crosslinking structure is introduced into the drug-containing adhesive layer 3, organic solvent is removed through drying and the crosslinking reaction is enhanced by in a heating environment for about 24 - 72 hr.

A method of laminating a support on an adhesive layer is not particularly limited.

For example, the container 11 is flipped vertically on a film used as a support to laminate the drug-containing adhesive layer 3 formed in the concave part 12 of the container 11 on a film, and the film is punched out in a given shape to form the support 2 to give a patch preparation, or a support 2 is laminated in the concave part 12 on the drug-containing adhesive layer 3 formed in the concave part 12 of the container 11 to give a patch preparation 1 with a given shape.

In the latter method, the support 2 is cut in advance into a shape covering the entirety of the drug-containing adhesive layer 3, and laminated on the surface of the drug-containing adhesive layer 3 formed in the concave part 12 of the container 11. As shown in Fig. 3, when a patch preparation 1 wherein the flat plane size of the support 2 is larger than that of the drug-containing adhesive layer 3 is manufactured, the support 2 is cut in advance into such size.

While the support 2 is not particularly limited, specifically, a single film of polyester (e.g., poly(ethylene terephthalate) (PET) etc.), nylon, polyvinyl chloride, polyethylene, polypropylene, ethylene-vinyl acetate copolymer, polytetrafluoroethylene, ionomer resin and the like, metal foil, or a laminate film wherein two or more kinds of films selected therefrom are laminated and the like can be mentioned. Of these, to improve adhesiveness (anchor property) of a support to an adhesive layer, it is preferable to use, as a support, a laminate film of a non-porous film made from the above-mentioned materials and the following porous film, and adhere the porous film side to the drug-containing adhesive layer 3. The thickness of the non-porous film is preferably 2 - 100 $\mu$m, more preferably 2 - 50 $\mu$m.

The porous film is not particularly limited as long as the anchor property to an adhesive layer is improved and, for example, paper, woven fabric, non-woven fabric (e.g., polyester (e.g., poly(ethylene terephthalate)(PET) and the like) non-woven fabric and the like), a single film of the above-mentioned film (e.g., polyester, nylon, Saran (trade name), polyethylene, polypropylene, ethylene-vinyl acetate copolymer, polyvinyl chloride, ethylene-ethyl acrylate copolymer, polytetrafluoroethylene, metal foil, poly(ethylene terephthalate) and the like, or a laminate film wherein two or more kinds of films selected therefrom are laminated and the like), which are mechanically perforated, and the like can be mentioned. Particularly, paper, woven fabric, non-woven fabric (e.g., polyester non-woven fabric, poly(ethylene terephthalate)non-woven fabric and the like) are preferable from the aspects of flexibility of the support. When the porous film is, for example, woven fabric or non-woven fabric, the fabric weight is preferably 5 - 30 g/m$^2$ to improve anchor property.

The laminate film for the support is manufactured by a known manufacturing method of laminate film such as dry lamination method, wet lamination method, extrusion lamination method, hot melt lamination method, coextrusion lamination method and the like.

While the thickness of the support is not particularly limited, it is generally 2 - 500 μm, preferably 2 - 200 μm, more preferably 10 - 50 μm. When it is less than 2 μm, handling property such as self-supporting property tends to decrease, and when it exceeds 500 μm, an unpleasant feeling (a feeling of stiffness) occurs during use of a patch preparation (during adhesion to the skin), and the followability to the skin tends to decrease.

A drug-containing adhesive layer 3 having a size and thickness of one patch preparation is formed in the concave part 12 of the container 11, and a support 2 is laminated on the drug-containing adhesive layer 3 to complete one patch preparation in the manufacturing method of the patch preparation in the present invention. Therefore, a step of cutting a drug-containing adhesive layer is not included in the manufacturing process. Consequently, a large physical stimulation is not applied to the drug-containing adhesive layer in the manufacturing process and, as is clear from the below-mentioned Examples, crystal of the drug is not precipitated in the drug-containing adhesive layer during preservation of each patch preparation after manufacturing.

In the patch preparation manufactured by the method of the present invention, the thickness of the drug-containing adhesive layer 3 is not particularly limited. The patch preparation manufactured by the method of the present invention shows sufficiently high drug releasability even without increasing the area and thickness of the drug-containing adhesive layer 3. Thus, the thickness of the drug-containing adhesive layer 3 is preferably about 10 - 500 μm, more preferably 15 - 300 μm, particularly preferably 20 - 250 μm, from the aspects of the handling property of the preparation.

In the patch preparation manufactured by the method of the present-invention, the drug to be contained in the drug-containing adhesive layer 3 is not particularly limited and may be the aforementioned drug. As a drug that permits more remarkable expression of the effect of the present invention method, a drug which is solid at room temperature (25°C) is preferable. The "drug which is solid at room temperature" means a drug having a melting point higher than 25°C in a DSC (differential scanning calorimeter) measurement. The "melting point" here is an extrapolation melting start temperature in a DSC curve measured according to JIS K 7121-1987. The extrapolation melting start temperature is a temperature at the intersection point between a straight line extending from the baseline on the low temperature side toward the high temperature side and a tangent line to the curve on the low temperature side of the meltpeak at the point of maximum gradient.

The drug for which a crystallization peak is not detected in a DSC measurement is preferable. Using such a drug, formation of the crystal of the drug in the drug-containing adhesive layer 3 immediately after manufacturing of a patch preparation (before preservation of the patch preparation) can be more certainly suppressed. Since a drug of which crystallization peak is not detected does not easily form its crystal structure, such a drug is assumed to be less apt to form a crystal in the adhesive layer. The crystallization peak here means an exothermic peak due to crystallization of a drug when DSC curve was measured according to JIS K 7121-1987. To be specific, 5 mg - 10 mg of a drug is measured in a measurement container, and the peak is measured by the following temperature program to give a DSC curve. The drug is maintained at a temperature lower by 50°C than the melting point of the drug for 10 min, heated at a temperature raise rate of 2°C per minute, and maintained at a temperature higher by 30°C than the melting point for 3 min. Thereafter, the temperature is decreased at a rate of 2°C per minute, and the drug is maintained at a temperature lower by 30°C than the glass transition temperature of the drug for 10 min, and then heated again at a rate of 2°C per minute to a temperature higher by 30°C than the melting point of the drug.

Examples of the drug for which a crystallization peak is not detected in a DSC measurement include miconazole, diltiazem, scopolamine, ketoprofen, indomethacin, capsaicin, ibuprofen, isosorbide dinitrate, diclofenac, rotigotine and the like.

A drug is dissolved in the drug-containing adhesive layer 3, and the weight concentration (wt%) of the drug in the drug-containing adhesive layer 3 is preferably 100 - 300%, more preferably 100 - 250%. When the ratio of the weight concentration to the saturation concentration [(weight concentration/saturation concentration)×100(%)] is less than 100%, sufficiently high skin permeation amount of the drug tends to be difficult to achieve, since the drug concentration of the adhesive layer is low. When it exceeds 300%, crystal formation of the drug tends to occur during the preservation of the patch preparation even if a physical stimulation is not applied to the adhesive layer after the manufacturing step of the patch preparation. The "weight concentration of the drug" here means an actual concentration (wt%) of the drug in the drug-containing adhesive layer 3 and the "saturation concentration of the drug" means the maximum amount of the drug that can be contained in the drug-containing adhesive layer at room temperature without precipitation of the drug. A saturation concentration of a drug can be determined by the following operation. Patch preparations with an adhesive layer having an incrementing weight concentration of the drug by 1 wt% are prepared, a liner on the adhesive layer of the respective patch preparations is detached, a small amount of a drug is left standing on the exposure face of the respective adhesive layers, and the liner is laminated again on the adhesive layer. The thus-manufactured patch preparations are stored in an incubator at 25°C for 6 months, and the size change of the drug left standing on the adhesive layer is observed every month under an optical microscope (magnification: 500-fold). When the drug size increases, the weight concentration of the drug is judged to have exceeded the saturation concentration, when the drug size decreases, the weight concentration of the drug is judged to be less than the saturation concentration, and when the drug size does not change, the weight concentration of the drug is judged to be near the saturation concentration.

This measurement method is based on the following facts. When the weight concentration of the drug is drastically smaller than the saturation concentration, the drug left standing dissolves in the adhesive layer to reduce the drug size, when the weight concentration of the drug is higher than the saturation concentration, crystal grows from the drug left standing to increase the drug size, and when the weight concentration of the drug is near the saturation concentration, the drug size does not change since dissolution and growth of the drug left standing are equilibrated.

For example, when the size of the drug left standing becomes small in a patch preparation having a weight concentration of the drug of 1 - 5 wt%, does not change in a patch preparation having a weight concentration of the drug of 6 - 7 wt%, and increases in a patch preparation having a weight concentration of the drug of 8 - 10 wt%, the saturation concentration of the drug in an adhesive layer is 7% (wt%). When the saturation concentration is judged to be less than 1% according to this method (when the size of the drug left standing increases in a patch preparation having a weight concentration of the drug of 1 wt%), a patch preparation having an incrementing weight concentration of the drug by 0.2 wt% within the range of 0 - 1 wt% is prepared, and evaluated in the same manner as above, and a saturation concentration is determined.

In conventional patch preparation manufactured by a method of obtaining a patch preparation with a desired flat planar shape by punching out a laminate sheet containing a drug-containing adhesive layer, wherein the weight concentration (wt%) of the drug in the adhesive layer is 100 - 300% of the saturation concentration (wt%) of the drug in the adhesive layer, when physical stimulation such as cutting (punching) is applied to the drug-containing adhesive layer, as shown in the below-mentioned Comparative Examples, crystal is precipitated in not less than 24 hr and within 6 months from the physical stimulation, irrespective of the presence or absence of crystallization peak of the drug detection. In contrast, in the manufacturing method of the patch preparation of the present invention, since a step of cutting the drug-containing adhesive layer is not included, physical stimulation is not applied to the drug-containing adhesive layer of the manufactured patch preparation. Therefore, as shown in the below-mentioned Examples, crystal of the drug is not formed (not precipitated) in the drug-containing adhesive layer during a 6-month preservation period at ambient temperature in a normal humidity environment without application of physical sitimulation to the drug-containing adhesive layer after manufacturing of the patch preparation. As used herein, ambient temperature means 25 ± 2°C and a noromal humidity means 60 ± 5%RH. Particularly, when a patch preparation is manufactured by the manufacturing method of the patch preparation of the present invention and using a drug for which a crystallization peak is not detected, formation of the crystal of the drug in the adhesive layer can be certainly suppressed.

In the patch preparation manufactured by the method of the present invention, when the drug-containing adhesive layer contains an organic liquid component, the content of the organic liquid component in the drug-containing adhesive layer is 5 - 70 wt%, more preferably 10 - 60 wt%, of the adhesive layer as 100 wt%. When the drug-containing adhesive layer contains a crosslinking agent, the content thereof is preferably 0.01 - 10 wt%, more preferably 0.05 - 5.0 wt%, of the adhesive layer as 100 wt%.

In the patch preparation manufactured by the method of the present invention, it is preferable that crystal of the drug not be formed in the drug-containing adhesive layer immediately after application of physical stimulation to the adhesive layer; however, further preservation results in the formation of crystal of the drug in the adhesive layer. Here, "immediately after" means 24 hr or less from the application of physical stimulation. When a crystal of the drug is formed immediately after application of physical stimulation on the adhesive layer, the concentration of the drug in the adhesive layer is too high. In this case, crystal formation of the drug has highly likely occurred when actually using the patch preparation. In case where a crystal of the drug is not formed in the adhesive layer when this further preserved, the concentration of the drug in the adhesive layer is low. As used herein, "when this further preserved" means not less than 24 hr and within 6 months after physical stimulation. In this case, a sufficient amount of the drug may not permeate through the skin when actually using the patch preparation.

In the present specification, "physical stimulation" means that the adhesive layer of a patch preparation is pierced through with a toughened and sharp-pointed part of a tool, for example, the adhesive layer of a patch preparation is pierced through with the edge of a cutter, the adhesive layer is cut with blades such as punching blade, cutter and the like.

The present applicant has found that crystal formation of the drug in a drug-containing adhesive layer can be suppressed by heating a patch preparation after packaging, and proposed same in Japanese Patent Application No. 2011-061515. This technique is associated with possible coloring of a drug-containing adhesive layer by heating. However, manufacturing of a patch preparation by the method of the present invention can also reduce the possibility of coloring of an adhesive layer.

[Manufacturing of patch preparation package]

When desired, the patch preparation manufactured by the method of the present invention can be packaged with a packaging material known per se. That is, patch preparation 1 in the container 11 or after taking out from the container 11 can be packaged with a packaging material such as resin film, metal foil and laminate film thereof and the like.
In addition, a blister package (blister pack) of a patch preparation can be manufactured by adhering a cap material to flange 13 formed on the periphery of the opening of the container 11 housing the patch preparation manufactured by the method of the present invention in concave part 12, thereby tightly sealing the container 11. The cap material that tightly seals the opening of the container 11 is not particularly limited as long as it is a sheet capable of heat sealing with the container 11. A material used as a cap material of a blister package (blister pack) in the field of patch preparations, specifically, resin film, metal foil, paper, and laminate films thereof and the like can be used. Examples
The present invention is explained in more detail in the following by referring to Examples, but is not limited by the following Examples. In the following description, "parts" fully means "parts by weight".

1. Preparation of polymer solution

(1) Preparation of acrylic polymer solution

Under an inert gas atmosphere, 2-ethylhexyl acrylate (75 parts), N-vinyl-2-pyrrolidone (22 parts), acrylic acid (3 parts) and azobisisobutyronitrile (0.2 parts) was subjected to solution polymerization in ethyl acetate at 60°C to give an acrylic polymer solution (polymer solid content: 28 wt%).

(2) Preparation of polyisobutylene polymer solution

High-molecular-weight polyisobutylene (viscosity average molecular weight 4000000, Oppanol(R) B200, manufactured by BASF, 22.0 parts as solid content), low-molecular-weight polyisobutylene (viscosity average molecular weight 55000, Oppanol(R) B12, manufactured by BASF, 38.0 parts as solid content), and alicyclic saturated hydrocarbon resin (softening point 140°C, ARKON(R) P-140, manufactured by Arakawa Chemical Industries, Ltd., 40.0 parts) were dissolved in toluene to give a polyisobutylene polymer solution (polymer solid content: 21 wt%).

2. Examples 1 - 7 and Reference Example 2

Starting materials were mixed in the formulations shown in Table 1 to give compositions for forming an adhesive layer.
A laminate sheet of a poly(ethylene terephthalate) (hereinafter to be abbreviated as "PET") sheet and an acrylonitrile resin sheet was formed to give a container having a concave part (depth: 15 mm) with a shape corresponding to a patch preparation to be manufactured (a support having 33 mm-square flat plane and a drug-containing adhesive layer with a thickness of 200 $\mu$m which covers the whole one surface of the support), and the inner surface of the concave part was peel-treated with a silicone release agent. The composition for forming an adhesive layer was poured into the concave part of this container such that the thickness after drying was 200 $\mu$m, and the composition was dried at 50°C for 5 min to sufficiently volatilize the solvent to form an adhesive layer. A PET film (thickness 25 $\mu$m) was adhered to the surface of the adhesive layer as a support to give a patch preparation.

3. Comparative Example 1

Starting materials were mixed in the formulation shown in Table 1 to give a composition for forming an adhesive layer, and the composition was applied to one surface of a PET film (thickness 75 $\mu$m) as a release liner such that the thickness after drying was 200 $\mu$m, and dried to form an adhesive layer. To the adhesive layer was adhered a PET film (thickness 25 $\mu$m) as a support, and the obtained laminate sheet was cut into a 33 mm-square flat plane shape to give a patch preparation.

4. Reference Example 1

Starting materials were mixed in the formulation shown in Table 1 to give a composition for forming an adhesive layer, and a patch preparation manufactured in the same manner as in Comparative Example 1 was packaged in a package container under an atmosphere of oxygen concentration not more than 3% and heated at 60°C for 12 hr to give a patch preparation.

5. Observation of crystal formation

The patch preparation of Examples 1 - 7, Comparative Example 1, and Reference Examples 1 and 2 was stored in an incubator at 25°C for 6 months. During the storage period for 6 months, the patch preparation was taken out from the incubator, and observed for crystal formation of the drug on the adhesive face of the adhesive layer and the inside thereof by visual observation and optical microscopic-observation (magnification: 500-fold).

A patch preparation that did not show crystal formation of the drug on the adhesive face of the adhesive layer and the inside thereof by visual and optical microscopic observation (magnification 500-fold) was evaluated as fine (O), a patch preparation that did not show crystal formation of the drug on the adhesive face of the adhesive layer and the inside thereof by visual observation but showed slight crystal formation by optical microscopic observation (magnification: 500-fold) was evaluated as good (△), and a patch preparation that showed clear crystal formation by visual observation was evaluated as not good (×).

6. Drug releasability

With regard to the patch preparations of Examples 1 - 7, Comparative Example 1, and Reference Examples 1 and 2, the drug releasability of the patch preparations was evaluated after storage of the patch preparations at 25°C for 6 months. The release test of the patch preparations was conducted according to U.S. Pharmacopeia 26, <724> Drug Release, Transdermal Delivery Systems-General Drug Release Standards, and a release solution was taken after 0.25 hr, 0.5 hr, 4 hr and 24 hr from the start of the test. The recovered solution was filtered with a membrane filter, quantified by high performance liquid chromatography (HPLC), and measured for the amount of the released drug. The release rate was calculated as the percentage of the amount of the drug (weight) released after a given time relative to the amount of the drug (weight) contained in the patch preparation before the drug release.

In addition, the drug release rate after a given time of the patch preparation stored at 25°C for 6 months and that of the patch preparation immediately after manufacturing were compared, and a patch preparation without change the drug release rate was evaluated as fine (O), and a patch preparation with change was evaluated as bad (x). Here, "without change the drug release rate" means that the difference between the drug release rate of a patch preparation stored at 25°C for 6 months and that of a patch preparation immediately after manufacturing was within 3% in all drug release rates that were taken after 0.25 hr, 0.5 hr, 4 hr and 24 hr from the start of the test.

7. Crystal formation of drug in adhesive layer due to physical stimulation

An adhesive layer of the patch preparation was pierced through with a cutter to give a physical stimulation. This was packed in packing containers and preserved at $25 \pm 2$°C and $60 \pm 5$%RH for 6 months. To determine whether a crystal of the drug was formed in the adhesive layer before physical stimulation, immediately after application of physical stimulation (not less than 0 hr and within 24 hr after physical stimulation), and when this was further preserved (not less than 24 hr and within 6 months after physical stimulation), the adhesive face of the adhesive layer and the inside thereof were observed visually and with an optical microscope. A patch preparation with crystal formation by visual observation and optical microscopic observation (magnification 500-fold) was evaluated as Y, a patch preparation without crystal formation by visual observation and optical microscopic observation (magnification 500-fold) was evaluated as N, and a patch preparation without crystal formation by visual observation but with crystal formation by optical microscopic observation (500-fold) was evaluated as S.

When a crystal of the drug is formed in the adhesive layer after application of physical stimulation (not less than 24 hr and less than 6 months after physical stimulation), the drug is dissolved and contained in the adhesive layer at a suitably high concentration. When a crystal of the drug is formed in 24 hr or less from the application of physical stimulation, the concentration of the drug in the adhesive layer is too high. In this case, crystal formation of the drug has highly likely occurred when actually using the patch preparation, which may prevent smooth use thereof. When a crystal of the drug is formed in 6 months or longer from the application of physical stimulation, the concentration of the drug in the adhesive layer is low. In this case, a sufficient amount of the drug may not permeate through the skin.

Table 1

| | Polymer (solid content) | | liquid component | | crosslinking agent | drug | | | | |
|---|---|---|---|---|---|---|---|---|---|---|
| | kind | amount (parts) | kind | amount (parts) | amount (parts) | name | concentration (wt%) | saturation concentration (wt%) | ratio of weight concentration to saturation concentration | crystallization peak |
| Example 1 | acrylic | 43.9 | IPM | 43.9 | 0.2 | diltiazem | 10 | 4 | 250% | not detected |
| Example 2 | acrylic | 42.4 | IPM | 42.4 | 0.2 | ISDN | 15 | 8 | 188% | not detected |
| Example 3 | acrylic | 44.4 | IPM | 44.4 | 0.2 | diclofenac | 11 | 9 | 122% | not detected |
| Example 4 | acrylic | 44.9 | ODO | 44.9 | 0.2 | ISDN | 10 | 4 | 250% | not detected |
| Reference Example 2 | acrylic | 43.4 | ODO | 43.4 | 0.2 | ISDN | 13 | 4 | 325% | not detected |
| Example 5 | acrylic | 40.4 | Coconard RK | 40.4 | 0.2 | ISDN | 19 | 11 | 173% | not detected |
| Example 6 | PIB | 69.0 | IPM | 30 | - | ISDN | 1 | 0.6 | 167% | not detected |
| Example 7 | acrylic | 47.4 | IPM | 47.4 | 0.2 | edaravone | 6 | 3 | 200% | detected |
| Comparative Example 1 | acrylic | 42.4 | IPM | 42.4 | 0.2 | ISDN | 15 | 8 | 188% | not detected |
| Reference Example 1 | acrylic | 42.4 | IPM | 42.4 | 0.2 | ISDN | 15 | 8 | 188% | not detected |

| | forming method | storage before physical stimulation | | relationship between physical stimulation and crystal formation | | |
|---|---|---|---|---|---|---|
| | | crystal observation (25°C 6 months) | released | before physical stimulation | immediately after physical stimulation | preservation after physical stimulation |
| Example 1 | composition is poured into container | ○ | ○ | N | N | Y |
| Example 2 | | ○ | ○ | N | N | Y |
| Example 3 | | ○ | ○ | N | N | Y |
| Example 4 | | ○ | ○ | N | N | Y |
| Reference Example 2 | | × | × | N | N | Y |
| Example 5 | | ○ | ○ | N | N | Y |
| Example 6 | | ○ | ○ | N | N | Y |
| Example 7 | | Δ | ○ | S | S | Y |
| Comparative Example 1 | cutting laminate sheet | × | × | N | N | Y |
| Reference Example 1 | cutting laminate sheet and heating | ○ | ○ | N | N | Y |

○: no crystal by visual observation and no crystal with microscope Δ: no crystal by visual observation and crystal present under microscope

×: crystal found by visual observation N: no crystal by visual observation and no crystal with microscope S: no crystal by visual observation and crystal present under microscope Y: crystal found by visual observation The terms in Table 1 mean as follows. PIB: polyisobutylene, IPM: isopropyl myristate, ODO: octyldodecanol, Coconard RK: caprylic acid triglyceride (manufactured by Kao Corporation), ISDN: isosorbide dinitrate

From the test results of Table 1, the following can be considered. The drug is present in a supersaturation amount in the adhesive layers of all the Examples, Comparative Example 1 and Reference Example 1, 2.

In Example 2, Comparative Example 1 and Reference Example 1, the formulation of the drug-containing adhesive layer is the same, but the manufacturing method of the patch preparation is different. In the patch preparation of Comparative Example 1, which was obtained by punching out a laminate sheet, crystal precipitated in the drug-containing adhesive layer, whereas in the patch preparation of Example 2, which was obtained by pouring a composition for forming an adhesive layer into a container having a concave part with a shape corresponding to the patch preparation, crystal did not precipitate. Since the drug concentration of the adhesive layer of Comparative Example 1 was supersaturated, it is considered that the physical stimulation applied to the adhesive layer during the punching processing formed a crystal core, and the crystal grew during preservation to a size sufficient to allow visual observation.

In the patch preparation of Example 2, crystal precipitation did not occur since the manufacturing process thereof does not contain a step of applying physical stimulation to the adhesive layer. While crystal precipitated in Comparative Example 1, it did not in Reference Example 1 containing a heating step after the manufacturing method of Comparative Example 1. It is considered that a crystal core was formed due to the physical stimulation during punching out a laminate sheet into a patch preparation, but the core was dissolved in the adhesive layer due to the heating step, and the crystal did not precipitate.

The patch preparations of Reference Example 2 and Comparative Example 1 failed to achieve sufficient permeability, since crystal precipitated during preservation after manufacturing and the drug release changed. When crystal precipitates, it induces patients' concern about the quality degradation, thereby making them hesitate to use the patch preparation. When the surface of the adhesive layer is covered with crystal, adhesion area decreases and adhesiveness to the skin becomes low, which prevents use of the patch preparation.

In Example 2 and Reference Example 1, the formulation of the drug-containing adhesive layer was the same but the manufacturing method was different, and crystal did not precipitate. Example 2 having no heating step of the patch preparation is considered to be advantageous since the apparatuses and time necessary for the heating step can be omitted. Furthermore, in Example 2, a composition for forming an adhesive layer is poured into a container with a shape of a patch preparation and dried to form a drug-containing adhesive layer. On the other hand, in Reference Example 1, a laminate sheet having an adhesive layer is punched out to manufacture a patch preparation. By comparison, Example 2 is economically more advantageous than Reference Example 1, since loss in the molding is small.

In addition, from the results of Examples 1 - 7, a patch preparation having a weight concentration of the drug in the adhesive layer of 100 - 300% relative to the saturation concentration can be manufactured by the manufacturing method of the present invention, regardless of the adhesive polymer, liquid component and drug contained in the patch preparation.

In Example 7, a drug with a detectable crystallization peak was used, and crystal of the drug was slightly observed. However, the amount of the crystal was sufficiently small and did not influence the adhesiveness to the skin during use. The crystal being slightly observed means that the crystal was not found by visual observation, but found under an optical microscope (x500).

**Claims**

1. A method of manufacturing a patch preparation comprising:

   pouring a composition for forming an adhesive layer into a container having a concave part with a shape corresponding to a patch preparation to be manufactured, wherein the composition contains a drug, an adhesive polymer and an organic solvent, wherein the depth of the concave part is not less than 110% of the thickness of the drug-containing adhesive layer of the patch preparation to be manufactured, and the difference between the depth of the concave part and thickness of the drug-containing adhesive layer (depth of concave part minus thickness of drug-containing adhesive layer) is not more than 20mm;
   removing the organic solvent from the composition through drying to form the drug-containing adhesive layer in the concave part; and
   laminating a support on the drug-containing adhesive layer to give the patch preparation comprising the support and the drug-containing adhesive layer formed on one surface of the support.

2. The method according to claim 1, wherein the concave part of the container has a peel-treated inner surface.

3. The method according to claim 1 or 2, wherein the drug is a solid at room temperature.

4. The method according to claim 1 or 2, wherein the drug does not have a crystallization peak in a DSC measurement.

**5.** The method according to claim 1 or 2, wherein the composition for forming an adhesive layer has a drug concentration adjusted such that the drug dissolved in the drug-containing adhesive layer formed in the concave part does not form a crystal immediately after application of physical stimulation to the adhesive layer, but forms a crystal when the patch preparation is further preserved.

**6.** A method of manufacturing a patch preparation package, comprising manufacturing a patch preparation according to the method according to any one of claims 1 to 5, housing the patch preparation in a container having a flange formed on the periphery of an opening, and adhering a cap material to the flange to tightly seal the container.

**Patentansprüche**

**1.** Verfahren zur Herstellung eines Pflasterpräparats, umfassend:

Gießen einer Zusammensetzung zur Bildung einer Kleberschicht in einen Behälter, der einen konkaven Teil mit einer Form, die einem herzustellenden Pflasterpräparat entspricht, aufweist, wobei die Zusammensetzung einen Wirkstoff, ein Klebepolymer und ein organisches Lösungsmittel enthält, wobei die Tiefe des konkaven Teils nicht weniger als 110% der Dicke der wirkstoffhaltigen Kleberschicht des herzustellenden Pflasterpräparats beträgt und die Differenz zwischen der Tiefe des konkaven Teils und der Dicke der wirkstoffhaltigen Kleberschicht (Tiefe des konkaven Teils minus Dicke der wirkstoffhaltigen Kleberschicht) nicht mehr als 20 mm beträgt; Entfernen des organischen Lösungsmittels aus der Zusammensetzung durch Trocknen unter Bildung der wirkstoffhaltigen Kleberschicht im konkaven Teil; und Laminieren eines Trägers auf die wirkstoffhaltige Kleberschicht, was das Pflasterpräparat ergibt, das den Träger und die auf einer Fläche des Trägers gebildete wirkstoffhaltige Kleberschicht umfasst.

**2.** Verfahren gemäß Anspruch 1, wobei der konkave Teil des Behälters eine trennbehandelte innere Oberfläche aufweist.

**3.** Verfahren gemäß Anspruch 1 oder 2, wobei der Wirkstoff bei Raumtemperatur ein Feststoff ist.

**4.** Verfahren gemäß Anspruch 1 oder 2, wobei der Wirkstoff bei einer DSC-Messung keinen Kristallisationspeak aufweist.

**5.** Verfahren gemäß Anspruch 1 oder 2, wobei die Zusammensetzung zur Bildung einer Kleberschicht eine Wirkstoffkonzentration aufweist, die so eingestellt ist, dass der Wirkstoff, der in der im konkaven Teil gebildeten wirkstoffhaltigen Kleberschicht gelöst ist, unmittelbar nach der Anwendung eines physikalischen Reizes auf die Kleberschicht keine Kristalle bildet, aber dann Kristalle bildet, wenn das Pflasterpräparat weiterhin aufbewahrt wird.

**6.** Verfahren zur Herstellung einer Pflasterpräparatpackung, umfassend das Herstellen eines Pflasterpräparats nach dem Verfahren gemäß einem der Ansprüche 1 bis 5, Unterbringen des Pflasterpräparats in einem Behälter mit einem Flansch, der am Rand einer Öffnung gebildet ist, und das Heften eines Verschlussmaterials auf den Flansch, um den Behälter dicht zu verschließen.

**Revendications**

**1.** Méthode de fabrication d'une préparation de patch comprenant :

le versement d'une composition pour former une couche adhésive dans un récipient comportant une partie concave ayant une forme correspondant à une préparation de patch à fabriquer, dans laquelle la composition contient un médicament, un polymère adhésif et un solvant organique, dans laquelle la profondeur de la partie concave ne fait pas moins de 110 % de l'épaisseur de la couche adhésive contenant le médicament de la préparation de patch à fabriquer, et la différence entre la profondeur de la partie concave et l'épaisseur de la couche adhésive contenant le médicament (profondeur de partie concave moins épaisseur de la couche adhésive contenant le médicament) ne fait pas plus de 20 mm ; le retrait du solvant organique de la composition par séchage afin de former la couche adhésive contenant le médicament dans la partie concave ; et la stratification d'un support sur la couche adhésive contenant le médicament afin de donner la préparation de patch comprenant le support et la couche adhésive contenant le médicament formée sur une surface du support.

2. Méthode selon la revendication 1, dans laquelle la partie concave du récipient a une surface intérieure traitée pour le décollement.

3. Méthode selon la revendication 1 ou 2, dans laquelle le médicament est un solide à température ambiante.

4. Méthode selon la revendication 1 ou 2, dans laquelle le médicament ne présente pas de pic de cristallisation lors d'une mesure (DSC Differential Scanning Calorimetry) par calorimétrie différentielle à balayage.

5. Méthode selon la revendication 1 ou 2, dans laquelle la composition pour former une couche adhésive a une concentration en médicament ajustée de sorte que le médicament dissous dans la couche adhésive contenant le médicament formée dans la partie concave ne forme pas de cristal juste après l'application d'une stimulation physique à la couche adhésive, mais forme un cristal quand la préparation de patch est davantage conservée.

6. Méthode de fabrication d'un emballage de préparation de patch, comprenant la fabrication d'une préparation de patch selon la méthode selon l'une quelconque des revendications 1 à 5, le logement de la préparation de patch dans un récipient ayant un rebord formé sur la périphérie d'une ouverture, et l'adhérence d'un matériau de protection sur le rebord afin de fermer hermétiquement le récipient.

# FIG. 1

# FIG. 2

# FIG. 3

1

2

3

1 3

1 2 B

1 2 A

1 2

1 1

# FIG. 4

1 1

1 3

1 2 B

1 2 A

1 2

W 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006513195 A
- JP 3566301 B
- JP 2011061515 A